# EUROPEAN PATENT APPLICATION

(11) **EP 4 124 653 A1**
(43) Date of publication of application: **01.02.2023**
(21) Application number: 21187983.8
(22) Date of filing: 27.07.2021
(51) Int. Cl.: C12M 1/26, B01D 15/12, C07K 1/36, C12M 1/36, C12M 1/00

(54) **METHOD FOR PRODUCING A BIOPRODUCT**

(71) Applicant: Sartorius Stedim Biotech GmbH, 37079 Göttingen (DE)
(72) Inventor: Saballus, Martin, 37249 Neu-Eichenberg (DE); Kampmann, Markus, 44149 Dortmund (DE)
(74) Representative: Gottschald Patentanwälte Partnerschaft mbB

(57) **Abstract**

The invention is directed to a method for producing a bioproduct using a bioprocess installation (1), wherein the bioprocess installation (1) comprises an electronic process control (2) and a bioprocess unit (3), wherein the bioprocess unit (3) comprises a receptacle (4), a clarification unit (5) with a centrifuge (6) and a chromatography unit (8) with a chromatograph (10), wherein cell broth obtained from the receptacle (4) is being lead through the clarification unit (5) and the chromatography unit (8) sequentially in a liquid stream (9), wherein the clarification unit (5) with its centrifuge (6) is being operated in a centrifugation cycle comprising centrifugation steps, such as loading-, washing- and discharging-steps, wherein the chromatography unit (8) with its chromatograph (10) is being operated in a chromatography cycle comprising chromatography steps, such as equilibration-, loading-, washing-, elution- and regeneration-steps, It is proposed that particle depletion between the liquid stream section leaving the clarification unit (5) and the liquid stream section leaving the chromatography unit (8) is less than 10% in particle concentration and that the execution of the steps assigned to the centrifugation cycle and the execution of the steps assigned to the chromatography cycle are at least partly being synchronized with each other by the electronic process control (2) in synchronization routines (13) based on assigned synchronization strategies (14).

## Description

The present invention relates to a method for producing a bioproduct using a bioprocess installation according to the general portion of claim 1, to an electronic process control for performing said method according to claim 15, to a computer program product for the electronic process control according to claim 17, to a computer-readable storage media, on which the computer program product is stored according to claim 18 and to a bioprocess installation with an electronic process control according to claim 19.

The term "bioprocess" presently represents any kind of biotechnological process, in particular biopharmaceutical processes. An example of such bioprocess is the use of a bioreactor to cultivate microorganisms or mammalian cells under given conditions, wherein a cell broth is transferred from the bioreactor to a downstream process.

The term "bioproduct" presently represents any kind of compound produced in such a bioprocess. Examples for such bioproducts are proteins, in particular antibodies, growth factors or hormones, metabolites or any other molecule as well as cells or their components.

The method in question for producing a bioproduct may be applied in various fields of biotechnology. High efficiency in this field has been driven by the increasing demand for bioproducts, such as biopharmaceutical drugs. The efficiency in this sense is regarding not only the cost-effectiveness of the components to be used but also the controllability of the processes connected thereto. The method in question relies on optimal interaction between the clarification of the cell broth of a bioprocess and its bioproduct purification to optimize controllability as well as time-effectiveness of the bioprocess.

On the one hand, chromatographs have to generate and maintain high and constant pressures for optimal purification results. On the other hand, chromatographs are in general prone to blocking by solid particles, which has a negative influence on chromatograph performance, leading to compromised purification results. Hence, it is common practice to interpose a filter directly upstream of a chromatograph, to prevent the chromatograph from blocking. However, fluids flow through a filter due to a pressure difference, from a high-pressure side upstream of the filter to a low-pressure side downstream of a filter. The filter itself is the cause of this pressure drop, also called filter resistance. This pressure drop occurs when frictional forces, caused by the resistance to flow, act on a fluid as it flows through the tube (DIN ISO 11057:2012-05 Emissionen aus stationären Quellen; Prüfverfahren für die Charakterisierung des Filtrationsverhaltens abreinigbarer Filtermedien (ISO 11057:2011); Beuth Verlag, Berlin. p. 9.). This filter resistance leads to a damping effect, delaying the transmission of fluid flow and fluid pressure. The main determinants of resistance to fluid flow are fluid velocity through the respective pipe and fluid viscosity. The pressure drop increases proportionally to the frictional shear forces within the piping network. These frictional shear forces increase with the number of interposed components, such as a filter.

Oversize particles and/or particle aggregates may form a filter cake (retentate), in particular when using static filtration where the filtrate is pultruded in the direction of the fluid flow, and block the filter lattice. In some cases, this blocking even prevents the fluid phase from crossing the filter, known as blinding (Sparks, Trevor; Chase, George (2015). Filters and Filtration Handbook (6th ed.)). Consequently, the concentration of potentially blocking solid particles and/or particle aggregates increases over time due to their accumulation on the filter lattice. As a result, the filter resistance increases continuously over time. It has been found that such changes in pressure lead to compromising process times, chromatograph performance and life span. Moreover, any changes in fluid pressure lead to non-uniform operational demands on the stationary phase of the chromatograph, leading to non-uniform separation results with limited reproducibility. Last, pressure variations can lead to disturbed sensor data acquisition, hence compromising the analytical outcome of chromatographs as well as quality control.

Alternatively, dynamic filtration can be used where the filtrate is pultruded orthogonally to the direction of the fluid flow. This creates shear stress that at least restricts the formation of the filter cake. As for static filtration, the driving force is also the pressure difference between the high-pressure side and the low-pressure side of the filter, the so-called transmembrane pressure (Handbook of Membrane Separations, Edited by Anil K.Pabby, Syed S.H.Rizvi, Ana aria Sastre, CRC Press). However, even such dynamic filtrations, like tangential flow- or cross-flow filtration, cannot completely prevent filters from blocking. Another problem that occurs during dynamic filtrations is that during the process, the transmembrane pressure might decrease due to an increase of filtrate viscosity, caused by the continuous concentration of the filtrate. Therefore filtration efficiency decreases and can be time-consuming for largescale processes. Another disadvantage is that tangential-flow filtrations comprise a low energy efficiency, since most of the energy invested in conveyance of the feed stream is lost via the retentate (Rautenbach, Robert: Membranverfahren Grundlagen der Modul- und Anlagenauslegung. Springer-Verlag, 1997).

One possible way of compensating for an increasing filter resistance would be to increase the fluid flow. However, in a bioprocess using cells for bioproduction, the fluid flow cannot be increased at will, due to increasing shear forces that could damage the cells and hence reduce the overall productivity of the process.

The known method for producing a bioproduct (WO 02/086135 A2), which is the starting point for the invention, makes use of a bioprocess installation comprising an electronic process control, a bioprocess unit with a receptacle, a clarification unit with a centrifuge and a chromatography unit with a chromatograph. In the method for producing a bioproduct, cell broth is obtained from the bioprocess unit and is lead through the clarification unit to clarify at least a part of the cells from the bioprocess unit. After completion of the clarification, the obtained product-containing supernatant can be passed through a filter, further removing solid particles, to potentially increase the lifetime of the chromatography unit. Finally, the supernatant can be passed through the chromatography unit for bioproduct purification. The resulting combination is of restricted efficiency in view of process times, process flexibility and adjustability in the mechanical setup as well as in the controllability of the overall process.

It is an object of the present invention to provide a method for producing a bioproduct, which increases the bioprocess efficiency and reproducibility with as little effort as possible.

The above-noted problem is solved for a method for producing a bioproduct with the features of the general part of claim 1 by the features of the characterizing part of claim 1.

The general concept underlying the invention is based on a direct connection of clarification unit and chromatography unit with hardly any solid/liquid separation, preferably without an interposed solid/liquid separation unit, in particular without any filtration unit, to enable synchronization of both units.

The term "solid/liquid separation" presently refers to processes that use the different physical and chemical properties of solid or liquid chemical substances mixed in a solution in order to separate them.

The above is facilitated by the elimination of solid/liquid separation units interposed between the clarification unit and chromatography unit of the bioprocess installation. Hence, a disruptive pressure drop caused by a filter can essentially be avoided, which gives the basis for a constant and undelayed fluid flow and/or fluid pressure. Interestingly, it has been found that this enables synchronization of the clarification unit and the chromatography unit, thereby even compensating for the lack of interposed solid/liquid separation units. First of all, this reduces dead times and process times, hence leading to an increased bioprocess efficiency with low effort. Second, it increases controllability as well as time-effectiveness, which is not only cost-efficient but also enables a simplified and more efficient control of the overall process.

However, it has been found that it is not enough to simply leave out the above noted solid/liquid separation units. Because without further measure, this would lead to chromatography column blocking during operation. Therefore it is proposed to enable the chromatography unit to let most solid particles pass through, such that those solid particles cannot lead to chromatography column blocking.

In detail, it is proposed, that particle depletion between the liquid stream section leaving the clarification unit and the liquid stream section leaving the chromatography unit is less than 10%, preferably less than 5%, in particle concentration and that the execution of the steps assigned to the centrifugation cycle and the execution of the steps assigned to the chromatography cycle is at least partly being synchronized with each other by the electronic process control in synchronization routines based on assigned synchronization strategies.

The term "particle depletion" relates to any reduction in the concentration of solid particles in a suspension, such as cells in media.

Claims 2 to 4 are directed to preferred embodiments of the connection between the clarification unit and chromatography unit. These measures solve the above-noted problem of a disturbing pressure drop caused by any interposed component damping and/or time-delaying fluid flow as well as fluid pressure transmission, such as a filter, and form the basis for synchronization of clarification unit and chromatography unit. Moreover, a direct connection between those units, without any interposed component, basically enables for control of the chromatography unit by the clarification unit, in particular by the centrifuge.

The preferred embodiments according to claims 5 to 8 refer to the different synchronization options, which enable increased flexibility regarding the choice of the respective synchronization strategy.

In claims 9 to 11, especially preferred embodiments regarding the respective steps to be synchronized are specified. These specifications allow for an optimized synchronization of the clarification unit and chromatography unit. This is particularly advantageous since buffers and/or washing liquids can be double-used by both involved units. This minimizes redundancies and saves resources, such as buffers or media. Another important advantage is that the product-containing supernatant can be first guided through the centrifuge before it is guided by liquid-synchronization to the chromatography unit, where it is directly loaded onto the chromatograph. In a similar way, a washing buffer could be used for both washing steps. This may be done, on the one hand, in order to rinse the cells with fresh buffer or growth media, and on the other hand, to wash out solid particles from the chromatograph. Fresh buffer helps the cells to remain healthy, while the washout of solid particles prolongs the lifetime of the chromatography unit. Moreover, a washout of solid particles solves the above-noted problem of accumulating particles that would otherwise lead to a disturbing increase in pressure drop over time.

The preferred embodiment according to claim 12 refers to the possible differences between a first and second culture environment and enables for increased flexibility regarding the choice of the respective entity, liquid properties and/or culture environment conditions of the first and second culture environment. This is particularly advantageous to adjust for the requirements of the cells so that they can be re-used for subsequent bioproduction.

Claims 13 to 15 are directed to preferred embodiments of the chromatography unit and the bioprocess installation. These measures minimize the risk of chromatography column blocking by solid particles and/or solid particle aggregates and gives flexibility regarding the kind of chromatographic separation technique.

According to a second independent teaching of claim 16, the whole bioprocess installation with a proposed electronic process control (claim 17) is claimed as such. All explanations given for the proposed method are fully applicable to the proposed bioprocess installation.

A third independent teaching according to claim 18 is directed to an electronic process control. The electronic process control is designed to perform the proposed method according to any one of the preceding claims. According to claim 19 the electronic process control comprises a data processing system for the realization of the proposed method, in particular the synchronization routine. All explanations given with regard to the first teaching are fully applicable to this third teaching.

According to another teaching of claim 20, which is of equal importance, a computer program product for the proposed data processing system is claimed as such. The computer program product is configured to realize the proposed method, in particular, to realize the above-noted routine. Again, all explanations given for the proposed method are fully applicable to the proposed computer program product.

According to another teaching of claim 21, which is of equal importance as well, a computer-readable storage media, on which the computer program is stored, is claimed as such. Again all explanations given for the proposed method are fully applicable to the proposed readable storage media.

In the following, an embodiment of the invention is explained with respect to the drawings. The drawings show
- Fig. 1: schematically a preferred embodiment of a proposed bioprocess installation, with which a proposed method is executable,
- Fig. 2: a flow chart representing the working principle of the proposed method according to fig. 1,
- Fig. 3: schematically preferred synchronization options regarding the respective start and/or end of the steps to be synchronized,
- Fig. 4: a table representing preferred various synchronization options regarding the respective steps to be synchronized according to the proposed method,

The proposed method for producing a bioproduct using a bioprocess installation 1 is preferably assigned to the upstream and downstream processes of a bioprocess, processing a liquid in the form of a cell broth for cell cultivation and/or bioproduction.

The term "liquid" is to be understood in a broad sense. It includes not only a pure liquid as such, but also emulsions and suspensions, e.g. a heterogeneous mixture of at least two different liquids or a heterogeneous mixture of solid particles and liquid.

The term "cell broth" is a suspension of cells and/or cell debris in media and describes the entirety of the cultivation medium and the respective organism cultured in the cultivation medium. Accordingly, the term "cell broth source" means any manufactured device or system capable of producing and/or storing cell broth.

The term "upstream process" involves all the steps related to cell bank, inoculum (seed train) development, media development, optimization of growth kinetics and the cultivation process itself as well as the corresponding inprocess control. The harvest of cells can be seen as both, part of upstreamand part of downstream processing.

The term "downstream process" involves all the steps related to the recovery and the purification of biosynthetic products, particularly biopharmaceuticals, from natural sources such as animal or plant tissue or cell broth, including the recycling of salvageable components and the proper treatment and disposal of waste.

In general, the cultivation of cells is currently used for the production of biopharmaceuticals, in particular proteins, such as human insulin, growth factors, hormones, vaccines, or antibodies, antibody derivatives, or the like. The products may as well be non-biopharmaceuticals, such as enzymes for food processing, laundry detergent enzymes, biodegradable plastics or biofuels. The focus of the present invention is on biopharmaceutical products secreted by the cells into the supernatant, such as antibodies or exosomes. Additionally or alternatively, the product can be the cells themselves, in particular mammalian cells including stem cells or immune cells such as CAR-T cells for the treatment of cancer.

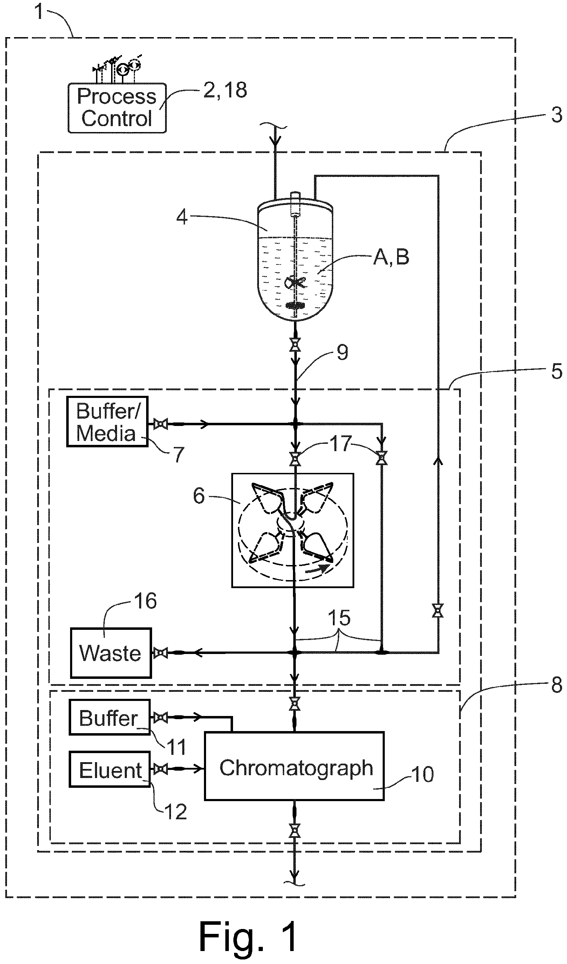

As shown in Fig. 1 to 4, according to all embodiments the proposed method for producing a bioproduct using a bioprocess installation 1 employs at least one electronic process control 2 and a bioprocess unit 3. The bioprocess unit 3 comprises a cell broth source with at least one receptacle 4 for cell broth including cultivation media and cells. Here and preferably, the at least one receptacle 4 is designed as a bioreactor (Fig. 1).

The term "cultivation media" means that the, preferably microbial or eukaryotic, cells used for the bioprocess grow in specially designed growth media, which supply the nutrients required by the respective organisms or cells. Varieties of media exist, but invariably contain at least a carbon source, a nitrogen source, water, salts, and micronutrients.

The term "bioreactor" in this context means any manufactured device or system that supports a biologically active environment by allowing monitoring and control of at least one parameter.

According to a preferred embodiment, the bioprocess unit 3 additionally comprises a second receptacle, which may be designed as a passive receptacle.

"Passive receptacle" means a receptacle without a feed line and/or without an electronic process control, which allows monitoring of at least one parameter within this passive receptacle. In contrast, "active receptacle" means a receptacle with a feed line and/or with an electronic process control, hence allowing for monitoring and/or controlling of at least one parameter within this active receptacle, such as a bioreactor.

According to Fig. 1, the bioprocess unit 3 comprises a clarification unit 5 with a centrifuge 6 for centrifugation of the cell broth.

"Centrifugation" is a term for sedimentation of particles in an artificially, by centrifugal forces created, gravitational field, wherein a significant reduction of separation time is achieved via large accelerating forces.

Here and preferably, the centrifuge 6 is designed as a fluidized-bed centrifuge for performing a continuous centrifugation process. Preferred setups of the centrifuge 6 are described in EP 2 485 846 A1, the contents of which are hereby incorporated by reference herein.

The centrifuge 6 comprises a rotor, which may be rotated around the centrifuge rotor axis by a, preferably electric, motor. The centrifuge revolution speed and the liquid pumping rate are adjustable by the electronic process control 2, with the aim to establish a fluidized bed of particles, such as cells or cell debris, in the centrifuge 6. A fluidized bed is achieved when the centrifugal force on a particle is equal to the opposing fluid flow force so that a zero net force is exerted on the particle.

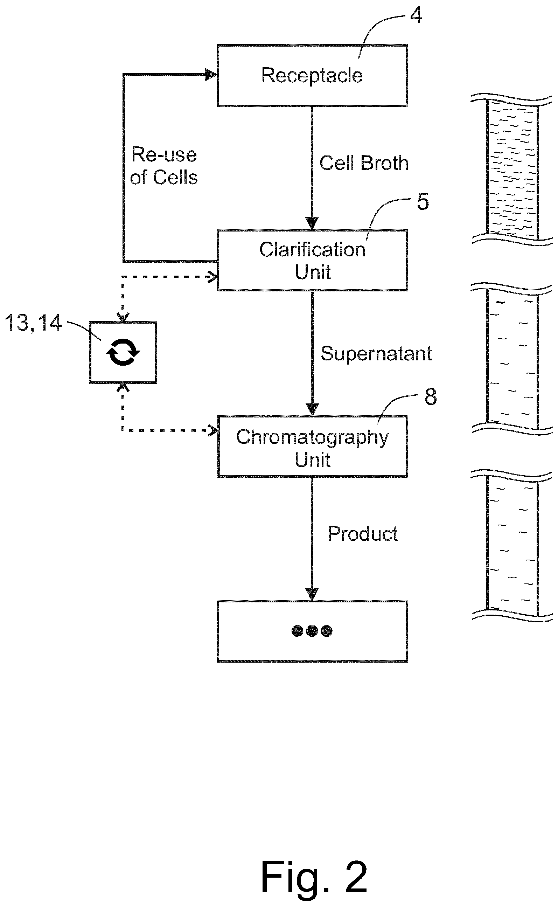

According to Fig. 2, the cell broth obtained from at least one receptacle 4 is being led through the clarification unit 5. The clarification unit 5 with its centrifuge 6 is being operated in a centrifugation cycle comprising centrifugation steps, such as loading-, washing- and discharging steps.

The centrifuge 6 can be operated in a forward operation for cell separation and/or cell washing. "Forward operation" means one out of two possible fluid flow directions in a centrifuge 6 and describes the operation leading to a separation of liquid and solid particles, such as media and cells. This "forward operation" allows, on the one hand, a washing of separated cells with buffer, preferably PBS buffer, or media, preferably cultivation media, further preferably enriched media, coming from a buffer/media receptacle 7, and/or, on the other hand, the clarification of the cell broth. The goal here is to clarify the liquid supernatant from solid particles such as cells, cell debris, etc., which solid particles are considered biomass. The product to be obtained in this forward operation is the supernatant of the cell broth containing a product of interest, e.g. a recombinant protein, in particular an antibody.

The term "enriched media" describes media that comprise higher concentrations of vitamins, growth factors, carbon-source, nitrogen-source and/or amino acid concentrations or the like and, preferably, allow the respective organism to grow at its maximum growth rate due to the optimized nutrient concentrations. Growth factors and trace nutrients are included in the media for organisms incapable of producing all of the vitamins they require. Inorganic nutrients, including trace elements such as iron, zinc, copper, manganese, molybdenum and cobalt are typically present in unrefined carbon and nitrogen sources but may have to be added when purified carbon and nitrogen sources are used.

The loading-step refers to a step of loading the respective centrifuge chamber in forward operation of the centrifuge 6 with cell broth to be centrifuged, obtained from the bioprocess unit 3.

The washing-step refers to a step of washing the respective centrifuge chamber in forward operation of the centrifuge 6 with media or buffer, coming from a buffer/media receptacle 7. This washing step preferably serves for a supply of fresh nutrients to the cells.

Alternatively, the centrifuge 6 can be operated in a backward operation. "Backward operation" means the second out of two possible fluid flow directions in the centrifuge 6 and describes the operation leading to a discharge of the separated solid particles, preferably cells. The product to be obtained in backward operation are the cells in the cell broth.

Hence, the discharging-step refers to the step assigned to the centrifuge 6, wherein in backward operation the centrifuge chamber is drained from solid particles, preferably cells. This discharging step can i.a. serve for transferring the cells back into a receptacle 4 for a re-use of cells in a subsequent bioprocess.

As can be seen in Fig. 2, the cell broth obtained from the bioprocess unit 3 is being lead through the clarification unit 5 and subsequently through a chromatography unit 8 sequentially in a liquid stream 9. The chromatography unit 8 further comprises at least one chromatograph 10, at least one buffer receptacle 11 and at least one eluent receptacle 12. The chromatography unit 8 with its chromatograph 10 is being operated in a chromatography cycle comprising chromatography steps, such as equilibration-, loading-, washing-, elution- and regeneration-steps.

The equilibration-step describes the step by which a system enters its state of equilibrium. In case of a chromatography unit 8, this refers to a filling of the respective chromatograph 10 with the respective buffer to be used for the subsequent bioproduct purification, until its entire volume is occupied by the respective buffer.

The loading-step refers to a step of loading the respective chromatograph 10 with product-containing supernatant obtained from the centrifuge 6 in order to bind the product to the chromatograph 10 and purify the bioproduct in a subsequent purification process by the chromatography unit 8.

The washing-step refers to a step of washing the chromatograph 10 with media or buffer, coming from a buffer receptacle 11. This washing step typically serves for flushing the chromatograph 10 to remove unspecifically bound compounds.

The elution-step describes the step of extracting one material from another, e.g. by washing with a solvent, such as water or imidazole. Here, it refers to the extraction of the bioproduct from the respective chromatograph 10 using an aqueous solution with pH- and/or conductivity gradients.

The regeneration-step describes the step of recovering a material or a system. In this particular context it refers to a re-equilibration and/or cleaning of the chromatograph 10, e.g. with NaOH solution.

It is particularly essential for the invention, that particle depletion between the liquid stream section leaving the clarification unit 5 and the liquid stream section leaving the chromatography unit 8 is less than 10%, preferably less than 5%, further preferably less than 2 % in particle concentration. Hence, remaining particles in the liquid stream 9 will be depleted by less than 10 % between the liquid stream section leaving the clarification unit 5 and the liquid stream section leaving the chromatography unit 8. In a preferred embodiment this includes a special construction of the chromatography unit 8, enabling the chromatography unit 8 to cope with particles and/or particle aggregates by being less prone to blocking. Also preferably, the chromatography unit 8 is set up for a washout of solid particles and/or particle aggregates.

According to the invention, the execution of the centrifugation steps and the execution of the chromatography steps are at least partly being synchronized with each other. This is controlled by the electronic process control 2 and organized in synchronization routines 13. These synchronization routines 13 are based on assigned synchronization strategies 14.

The term "synchronization" means the bidirectional temporal coordination of events to operate a system in unison.

In the embodiments according to Fig. 1 and 2, here and preferably, the chromatography unit 8 receives the liquid stream section leaving the clarification unit 5 in an unfiltered manner.

The term "unfiltered" means here to omit any kind of separation process, between the liquid stream section leaving the clarification unit 5 and the liquid stream section leaving the chromatography unit 8, leading to a solid particle depletion of more than 10 %, preferably of more than 5 %, further preferably of more than 2 %. Exemplary separation processes are filtration, in particular dead-end filtration or cross-flow filtration, centrifugation or reverse osmosis.

In a preferred embodiment, the chromatography unit 8 comprises a feed input, which is directly connected to an outlet of the centrifuge 6. According to this embodiment, no additional device needs to be interposed in between the centrifuge 6 and the chromatography unit 8 as can be seen in Fig. 1 and Fig. 2.

In another preferred embodiment, the liquid stream 9 is at least temporarily continuous. Hence, the chromatography unit 8, in particular the chromatograph 10, can be controlled by controlling the clarification unit 5, in particular the centrifuge 6.

"Continuous" means here that the liquid stream 9 coming from the bioprocess unit 3, passing the clarification unit 5 and finally the chromatography unit 8 is, at least temporarily, one continuous stream, without being stopped, delayed or the like.

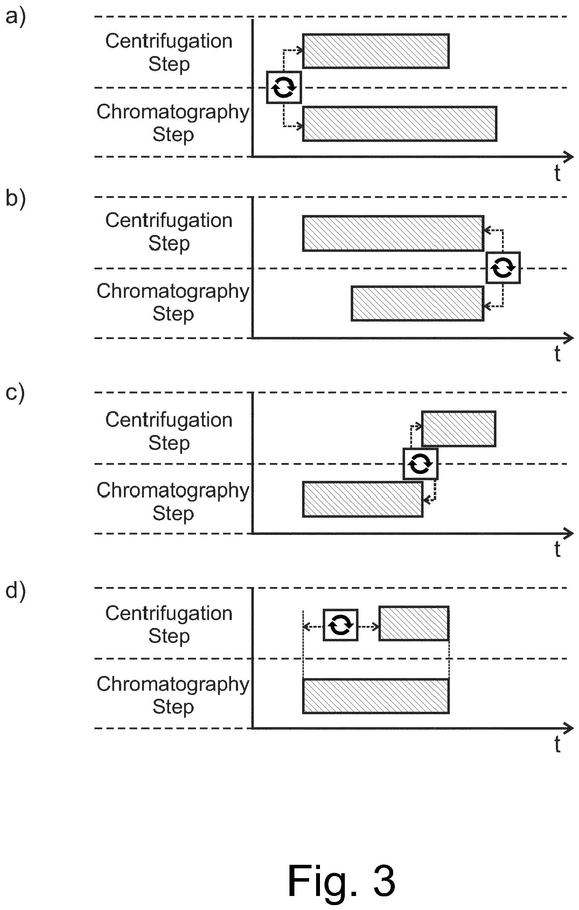

According to another preferred embodiment, as can be seen in Fig. 3, for at least one synchronization routine 13, the synchronization strategy 14 represents a time-synchronization. According to the time-synchronization, at least one centrifugation step stays in a predefined time-wise relation to at least one chromatography step.

The term "time-synchronization" means here the bidirectional coordination of events according to a predefined time-wise relation between the respective centrifugation step and the respective chromatography step.

In another embodiment, according to the time-synchronization and as can be seen in Fig. 3, the steps to be synchronized are being started simultaneously (Fig. 3a). Additionally or alternatively, the steps to be synchronized are being ended simultaneously (Fig. 3b). In yet another embodiment, the steps to be synchronized are being started and/or ended simultaneously, or with a predefined delay (Fig. 3c). The steps to be synchronized can be any centrifugation step or any chromatography step.

The term "predefined" means here, that it can be defined in advance, preferably by the user.

According to an especially preferred embodiment, according to the time-synchronization, one of the steps to be synchronized is being started in dependence of the start of the respective other steps. Alternatively, one of the steps to be synchronized is being started in dependence of the end of the respective other steps (Fig. 3d).

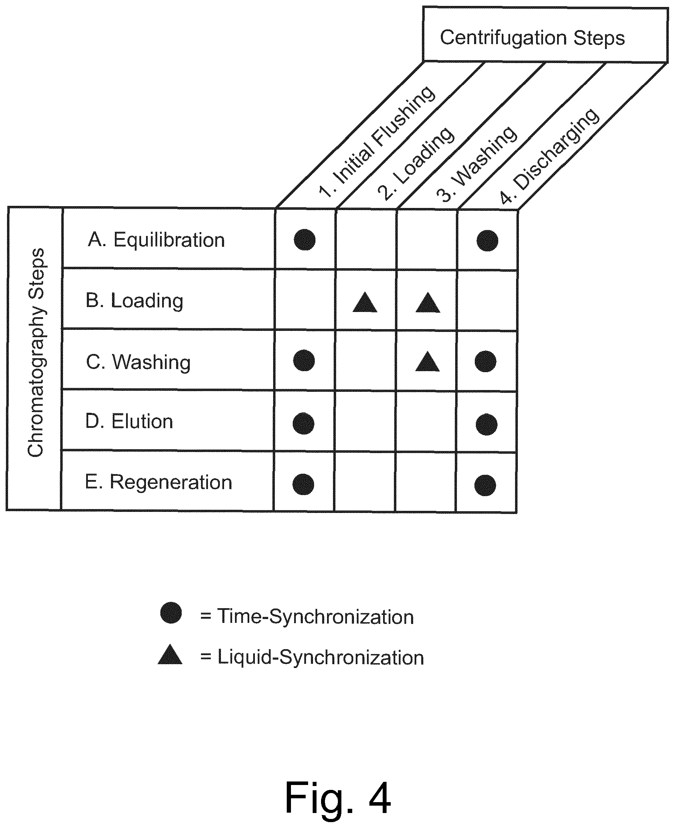

Preferably, according to Fig. 4, for at least one synchronization routine 13, the synchronization strategy 14 represents a liquid synchronization. This is preferably in addition to a time-synchronization. According to the liquid-synchronization, in at least one centrifugation step, the liquid is transferred to the chromatography unit 8.

The term "liquid-synchronization" means here the bidirectional coordination of events according to a predefined liquid-wise relation between the respective centrifugation step and the respective chromatography step. "Liquid-wise" preferably means a fluidic connection between the involved components. As an example, liquid passing the clarification unit 5, in particular the centrifuge 6, can be directly guided to the chromatography unit 8, in particular the chromatograph 10, in order to double-use one liquid for both units 5, 8. This can be achieved either by using a separate liquid line 15 or the same. The liquid can be the supernatant, buffer, growth media or the like.

In another especially preferred embodiment according to Fig. 4, for at least one synchronization routine 13, according to the synchronization strategy 14, the loading-step of the centrifugation cycle is synchronized with the loading-step of the chromatography cycle. It is especially noteworthy that the product-containing supernatant resulting from centrifugation in forwarding operation and exiting the centrifuge 6 can directly be loaded onto the chromatograph 10 by liquid-synchron ization.

According to another especially preferred embodiment according to Fig. 4, for at least one synchronization routine 13, according to the synchronization strategy 14, the washing-step of the centrifugation cycle is synchronized with the loading step of the chromatography cycle. Preferably, the synchronization strategy 14 represents a liquid synchronization. This is particularly interesting since it takes dead volumes into account. Thereby, as much of the product-containing supernatant as possible is loaded onto the chromatograph 10.

Additionally or alternatively, the washing step of the centrifugation cycle is synchronized with the washing step of the chromatography cycle. It is especially interesting that the liquid used for the washing-step of the centrifugation cycle and exiting the centrifuge 6 can directly be guided to the chromatograph 10 for realizing the washing-step of the chromatography cycle, set up for a washout of unspecifically bound compounds and solid particles. Preferably, the synchronization strategy 14 represents a liquid synchronization. Hence, the same liquid can be used for both, the washing step of the centrifugation cycle as well as for the washing step of the chromatography cycle. The liquid can be any buffer or media or the like, which is (bio)compatible with, on the one hand, the bioproduct-producing cells inside the centrifuge 6, and, on the other hand, the stationary phase of the chromatograph 10. It is especially preferred, that at least one liquid is a double-used liquid, used for the centrifugation cycle and the chromatography cycle as well.

In another embodiment, according to Fig. 4, for at least one synchronization routine 13, according to the synchronization strategy 14, the discharging-step of the centrifugation cycle is synchronized with any one of the equilibrations -, washing-, elution- and/or regeneration-step of the chromatography cycle. Here and preferably, the synchronization strategy represents a time-synchronization.

Here and preferably, according to Fig. 1 and Fig. 2, out of a first culture environment A established by the bioprocess unit 3, the cell broth is transferred to the centrifuge 6. In backward operation of the centrifuge 6, at least part of the discharged cells is being transferred into a separate, second culture environment B. Culture environment B is different from the first culture environment A and established for subsequent cell cultivation and/or bioproduction.

Additionally or alternatively, the second culture environment B differs from the first culture environment A with respect to liquid properties, e.g. choice of media, in particular choice of enriched media, comprising i.a. optimized carbon-source concentrations, optimized nitrogen-source concentrations, optimized amino acid concentrations and/or optimized growth factor concentrations, and/or the liquid volume used in the bioprocess.

Further additionally or alternatively, the second culture environment B differs from the first culture environment A with respect to culture environment conditions, such as choice of gas concentrations, in particular, oxygen and/or carbon dioxide concentrations, and/or choice of pH, and/or choice of temperature or the like. Exemplary amino acid-sources can be peptone or tryptone in concentrations of 0.5 % to 2 %. Exemplary carbon-sources can be glucose or sucrose in concentrations of 0.1 % to 3 %.

It is also preferred, that the second culture environment B is the first culture environment A, after having, preferably completely, exchanged the cultivation media. In these embodiments, according to Fig. 1 and Fig. 2, the discharged cells can be transferred for re-use of the cells for cell cultivation and/or bioproduction, after the cultivation media and/or cells have been, at least partially, removed. Thereby a backmixing of consumed and fresh cultivation media and/or cells is individually controllable.

Preferably, the resulting ratio of consumed to fresh liquid is individually adjustable. Additionally or alternatively, the second culture environment B preferably provides culture environment conditions thereby favouring cell growth and/or bioproduction. The term "Favoring cell growth and/or bioproduction" means an enhancement and support of at least one parameter either mirroring the cells' vitality and/or the cells' productivity, including but not limited to growth rate, viability (i.e. a percentage of living cells), productivity, oxygen uptake rate and/or biomass production rate.

In another preferred embodiment, the second culture environment B may be provided by the receptacle 4, which, for establishing the second culture environment B, is being brought to a cell-free and/or a liquid-free state in a preparation phase. In a preferred embodiment, the preparation phase consists of at least one workflow step to establish conditions for cell cultivation and/or bioproduction.

The above workflow step can be at least one out of the group of media preparation, (complete) emptying, cleaning, maintaining, sterilizing, replacing and/or loading the receptacle with media, preferably enriched media. "Media preparation" describes the act of composing the media to be used in the bioprocess, in particular the calculations of the specific component ratios and the actual manufacture of the media.

In a preferred embodiment, the workflow step may be at least partial, preferably complete, emptying of the receptacle 4. In another example, the receptacle 4 needs to be at least partially, preferably completely, cleaned. In a further example, the workflow step may be the maintenance of the receptacle 4, in order to ensure an optimal bioprocess, or a replacing of the receptacle 4, in particular, if the receptacle 4, or in particular any receptacle of the bioprocess unit 3, is designed as a single-use device. Another possible workflow step could be the loading of the receptacle 4 with media, in particular enriched media, as described above. For this procedure, the media needs to be filled into the receptacle, first. Any cultivation media can be used, preferably any enriched media, which comprises higher nutrient concentrations etc. Further preferably, these media do not constitute the limiting factor for cell cultivation and/or bioproduction. Here and preferably, and just as an example 4Cell^{®} CHO Media, NutriStem^{®} hPSC XF Medium, RoosterNourish^{™}-MSC, Gibco Cell Culture Medium, DMEM, IMDM, or the like might be used, depending on the bioprocess of choice. Further preferably, media that lead to optimal cell growth and proliferation rates, healthy physiology, morphology and/or proper gene expression are used.

Preferably, the second culture environment B differs from the first culture environment A with respect to the structural entity establishing the respective culture environment A, B. In these embodiments, the second culture environment B is provided by a second receptacle, which is separate from the first receptacle 4. This allows for a transfer of cells from the first receptacle 4 to a second receptacle. Preferably, when starting operation of the proposed bioprocess installation 1, the second receptacle is free from cell broth of the first receptacle 4. The entity establishing the second culture environment B can, for instance, be a second receptacle, such as a storage vessel or a second bioreactor.

As indicated by Fig. 1, the clarification setup 5 comprises a waste receptacle 16, preferably designed as a waste vessel. Here and preferably, in the backward operation of the centrifuge 6, the liquid, preferably the solid particles in discharging liquid, further preferably the cells in discharging liquid, is/are transferred to the second culture environment B or the waste receptacle 21, controlled by the electronic process control 2.

The chromatography unit 8 comprises a stationary phase and the stationary phase preferably consists of beads. These, preferably spherical beads comprise a size of at least 250 µm in diameter, further preferably of at least 350 µm in diameter.

According to another preferred embodiment, the stationary phase consists of at least one membrane, preferably a plurality of stacked membranes, or a monolith. According to this embodiment, the at least one membrane and the monolith comprise a multitude of pores. These pores comprise a pore size of more than 20 µm, preferably of more than 40 µm..

Hence, these embodiments of the stationary phase will prevent particles with a size of up to 20 µm, preferably with a size of up to 40 µm, from penetrating and eventually blocking the pores of the stationary phase. The material of the stationary phase is preferably agarose, polyamide, polysulfone, polyether sulfone (PES), polyvinylidene fluoride (PVDF), cellulose, cellulose acetate, silicone, polycarbonate, polyacrylonitrile (PAN), polyethylene (PE), polypropylene (PP), phenyl or the like.

It is particularly preferred, that these embodiments concerning the stationary phase of the chromatography unit 8 are used for affinity chromatography, in particular Protein A affinity chromatography, ion-exchange chromatography (IEX), such as anion exchange chromatography (AEX) or cation exchange chromatography (CEX), hydrophobic interaction chromatography (HIC), sizeexclusion chromatography (SEC) or any other type of chromatography techniques. Moreover, these chromatography techniques can be operated in axial flow or radial flow as well as additionally combined with simulated moving bed chromatography, enabling for multiple parallel chromatography cycles. Therefore, the chromatography unit comprises at least one chromatograph. This at least one chromatograph comprises at least one chromatography column, preferably a multitude of chromatography columns.

In another especially preferred embodiment, the bioprocess unit 3 is designed to cope with particle sizes between 10 µm and 100 µm, preferably between 15 µm and 80 µm, further preferably between 20 and 60. Consequently, solid particles, in particular cells, with a size up to 20 µm in diameter may be lead through the bioprocess unit 3, preferably in the chromatography unit 8, without causing any blocking of the chromatography unit 8, in particular of the chromatograph 10.

Preferably, at least one component of the bioprocess unit 3, preferably at least one component of the receptacle 4, the clarification unit 5, in particular the centrifuge 6, and/or the chromatography unit 8, further preferably all components of the bioprocess unit 3, are designed as single-use components.

According to another, independent teaching, a bioprocess installation 1 is claimed as such, with an electronic process control 2 and a bioprocess unit 3. The bioprocess unit 3 comprises at least one receptacle 4, a clarification unit 5 with a centrifuge 6 and a chromatography unit 8 with a chromatograph 10. Cell broth obtained from the receptacle 4 is first being lead through the clarification unit 5. Subsequently, the cell broth is being led through the chromatography unit 8. The passage of both units 5, 8 is conducted sequentially in a liquid stream 9. The clarification unit 5 with its centrifuge 6 is being operated in a centrifugation cycle comprising centrifugation steps, such as such as loading-, washing- and discharging steps. On the other hand, the chromatography unit 8 with its chromatograph 10 is being operated in a chromatography cycle comprising chromatography steps, such as equilibration-, loading-, washing-, elution- and regeneration-steps.

It is essential, that particle depletion between the liquid stream section leaving the clarification unit 5 and the liquid stream section leaving the chromatography unit 8 is less than 10%, preferably less than 5%, further preferably less than 2 % in particle concentration and that the execution of the steps assigned to the centrifugation cycle and the execution of the steps assigned to the chromatography cycle are at least partly being synchronized with each other by the electronic process control 2 in synchronization routines 13 based on assigned synchronization strategies 14.

The electronic process control 2 is preferably designed to perform the proposed method by controlling the bioprocess unit 3, the clarification unit 5 with its centrifuge 6 and/or the chromatography unit 8 with its chromatograph 10. The electronic process control 2 may be realized as a central unit controlling all or at least most of the components of the bioprocess installation 1. The electronic process control 2 may also be realized in a decentralized structure, comprising a number of decentralized units. In some embodiments, the at least one electronic process control 2 directs the opening and closing of one or more valve(s) 17 (Fig. 1), the rotational speed of the rotor, either directly or via a motor, and/or the flow direction and/or velocity of the fluid and/or particles from a receptacle 4, such as a bioreactor.

Such an electronic process control 2 comprises for instance at least one digital control unit (DCU) and/or at least one multi fermenter control system (MFCS), which comprises a local processor unit and a local data storage itself. The MFCS also provides a centralized process management system, dispatching requests to the digital control unit. Additionally or alternatively, such an electronic process control 2 comprises preferably a computer, and/or a server, and/or a smartphone or the like. Here and preferably, the electronic process control 2 is individually adjustable and/or programmable and/or comprises at least one microprocessor, on which software may be run. All explanations given before are fully applicable to this teaching.

According to another, independent teaching, an electronic process control of the bioprocess installation 1 is claimed as such. Again, reference is made to all explanations given before.

It is essential, that the electronic process control 2 is designed for performing the proposed method by controlling the clarification unit 5, in particular the centrifuge 6, and/or the chromatography unit 8, in particular the chromatograph 10.

Preferably, the electronic process control 2 comprises a data processing system 18 for the realization of the above-noted method, preferably comprising a local data storage and a local processor unit.

Finally, independent teachings are directed to a computer program product for the electronic process control 2 and to a computer-readable storage media, on which the computer program product is stored, preferably in a non-volatile manner.

## Claims

1. Method for producing a bioproduct using a bioprocess installation (1), wherein the bioprocess installation (1) comprises an electronic process control (2) and a bioprocess unit (3), wherein the bioprocess unit (3) comprises a receptacle (4), a clarification unit (5) with a centrifuge (6) and a chromatography unit (8) with a chromatograph (10), wherein cell broth obtained from the receptacle (4) is being lead through the clarification unit (5) and the chromatography unit (8) sequentially in a liquid stream (9), wherein the clarification unit (5) with its centrifuge (6) is being operated in a centrifugation cycle comprising centrifugation steps, such as loading-, washing- and discharging-steps, wherein the chromatography unit (8) with its chromatograph (10) is being operated in a chromatography cycle comprising chromatography steps, such as equilibration-, loading-, washing-, elution- and regeneration-steps,
**characterized in**
**that** particle depletion between the liquid stream section leaving the clarification unit (5) and the liquid stream section leaving the chromatography unit (8) is less than 10% in particle concentration and that the execution of the steps assigned to the centrifugation cycle and the execution of the steps assigned to the chromatography cycle are at least partly being synchronized with each other by the electronic process control (2) in synchronization routines (13) based on assigned synchronization strategies (14).

2. Method according to claim 1, **characterized in that** the chromatography unit (8) receives the liquid stream section leaving the clarification unit (5) in an unfiltered manner.

3. Method according to claim 1 or 2, **characterized in that** the chromatography unit (8) comprises a feed input, which is directly connected to an outlet of the centrifuge (6).

4. Method according to any of the preceding claims, **characterized in that** the liquid stream (9) is at least temporarily continuous.

5. Method according to any one of the preceding claims, **characterized in that** for at least one synchronization routine (13), the synchronization strategy (14) represents a time-synchronization and that according to the time-synchronization, at least one centrifugation cycle stays in a predefined time-wise relation to at least one chromatography cycle.

6. Method according to claim 5, **characterized in that** according to the time-synchronization, the cycles to be synchronized are being started and/or ended simultaneously or with a predefined delay.

7. Method according to claim 5 or 6, **characterized in that** according to the time-synchronization, one of the cycles to be synchronized is being started in dependence of the start or the end of the respective other cycles.

8. Method according to any one of the preceding claims, **characterized in that** for at least one synchronization routine (13), the synchronization strategy (14), preferably in addition to representing a time-synchronization, represents a liquid-synchronization, and that according to the liquid-synchronization, in at least one centrifugation cycle, liquid is transferred to the chromatography unit (8).

9. Method according to any one of the preceding claims, **characterized in that** for at least one synchronization routine (13), according to the synchronization strategy (14), the loading-step of the centrifugation cycle is synchronized with the loading-step of the chromatography cycle.

10. Method according to any one of the preceding claims, **characterized in that** for at least one synchronization routine (13), according to the synchronization strategy (14), the washing-step of the centrifugation cycle is synchronized with the loading- and/or washing-step of the chromatography cycle.

11. Method according to any one of the preceding claims, **characterized in that** for at least one synchronization routine (13), according to the synchronization strategy (14), the discharging-step of the centrifugation cycle is synchronized with any one of the equilibrations -, washing-, elution- and/or regeneration-step of the chromatography cycle.

12. Method according to any one of the preceding claims, **characterized in that** out of a first culture environment (A) established by the bioprocess unit (3), the cell broth is transferred to the centrifuge (6), that in a backward operation of the centrifuge (6), at least part of the discharged cells is being transferred into a separate, second culture environment (B) different from the first culture environment (A) for subsequent cell cultivation and/or bioproduction.

13. Method according to any one of the preceding claims, **characterized in that** the chromatography unit (8) comprises a stationary phase, that the stationary phase consists of, preferably spherical, beads and that the beads comprise a size of at least 250 µm in diameter, further preferably of at least 350 µm in diameter.

14. Method according to any one of the preceding claims, **characterized in that** the chromatography unit (8) comprises a stationary phase, that the stationary phase consists of at least one membrane, preferably a plurality of stacked membranes, or a monolith and that the stationary phase comprises a multitude of pores.

15. Method according to any one of the preceding claims, **characterized in that** the bioprocess unit (3) is designed to cope with particle sizes between 10 µm and 100 µm, preferably between 20 µm and 40 µm.

16. Bioprocess installation (1) with an electronic process control (2) and a bioprocess unit (3), wherein the bioprocess unit (3) comprises a receptacle (4), a clarification unit (5) with a centrifuge (6) and a chromatography unit (8) with a chromatograph (10), wherein cell broth obtained from the receptacle (4) is being led through the clarification unit (5) and the chromatography unit (8) sequentially in a liquid stream (9), wherein the clarification unit (5) with its centrifuge (6) is being operated in a centrifugation cycle comprising centrifugation steps, such as loading-, washing- and discharging-steps, wherein the chromatography unit (8) with its chromatograph (10) is being operated in a chromatography cycle comprising chromatography steps, such as equilibration, loading-, washing-, elution- and regeneration-steps,
**characterized in**
**that** particle depletion between the liquid stream section leaving the clarification unit and the liquid stream section leaving the chromatography unit (8) is less than 10% in particle concentration and that the execution of the steps assigned to the centrifugation cycle and the execution of the steps assigned to the chromatography cycle are at least partly being synchronized with each other by the electronic process control (2) in synchronization routines (13) based on assigned synchronization strategies (14).

17. Bioprocess installation according to claim 16, **characterized in that** the electronic process control (2) is designed for performing the method according to any one of the claims 1 to 15 by controlling the clarification unit (5), in particular the centrifuge (6), and/or the chromatography unit (8), in particular the chromatograph (10).

18. Electronic process control of the bioprocess installation (1) according to claim 16 or 17, **characterized in that** the electronic process control (2) is designed for performing the method according to any one of the claims 1 to 15 by controlling the clarification unit (5), in particular the centrifuge (6), and/or the chromatography unit (8), in particular the chromatograph (10).

19. Electronic process control according to claim 18, **characterized in that** the electronic process control (2) comprises a data processing system (18) for realizing the method according to claim 1 to 15.

20. Computer program product for the electronic process control (2) according to claim 18 or 19.

21. Computer-readable storage media, on which the computer program product according to claim 20 is stored, preferably in a non-volatile manner.
